# EUROPEAN PATENT APPLICATION

(11) **EP 1 452 587 A1**
(43) Date of publication of application: **01.09.2004**
(21) Application number: 02781773.3
(22) Date of filing: 15.11.2002
(51) Int. Cl.: C12N 5/06, A61F 2/02

(54) **STEM CELLS ORIGINATING IN SALIVARY DUET EPITHELIAL CELLS AND USE THEREOF**

(30) Priority: 15.11.2001 JP 2001350541
(71) Applicant: Bios Research Institute Inc., Hiratsuka-shi, Kanagawa 254-0031 (JP); Endo, Fumio, Kumamoto-shi, Kumamoto 862-0941 (JP)
(72) Inventor: ENDO, Fumio, Kumamoto-shi, Kumamoto 862-0941 (JP); OKUMURA, Kenji, Kumamoto-shi, Kumamoto 862-0950 (JP); NAKAMURA, Kimitoshi, Kumamoto-shi, Kumamoto 862-0941 (JP)
(74) Representative: Walton, Seán Malcolm
(86) International application number: PCT/JP2002/011914
(87) International publication number: WO 2003/042375

(57) **Abstract**

A stem cell which can be differentiated to a hepatic cell, a pancreas cell or to a salivary gland cell is disclosed. The stem cell according to the present invention is originated from salivary gland duct epithelium, which can be differentiated to an alpha-fetoprotein-positive cell, an albumin-positive cell, an amylase-positive cell, an insulin-positive cell and a glucagon-positive cell by culture *in vitro*. By transplanting the stem cells according to the present invention, organs such as liver can be regenerated.

## Description

### Technical Field

The present invention relates to a stem cell originated from salivary gland duct epithelium and use of the same as a cell to be transplanted for regeneration of an organ.

### Background Art

Stem cells which are potentially useful for regenerative medicine are now widely studied. Representative stem cells which have been reported include mesenchymal stem cells, neural stem cells, hematopoietic stem cells and pancreatic stem cells.

Mesenchymal stem cells were separated from human adult bone marrow fluid (Pittenger, M. F. et al., Science 284, 143(1999)). These cells are capable of being differentiated to fat cells, cartilage cells, and bone cells *in vitro.* As for neural stem cells (Gage, P.H., science 287, 1433-1438(2000)), separation of neural stem cells from adult central nerve system was first reported in 1992, and separation of stem cells capable of being differentiated to nerve cells, which are originated from adult dermis, was reported (Toma, J.G.et al., Nature Cell Biology, 3, 778-784(2001)).

Although a number of studies on hematopoietic stem cells have been made, reports on their differentiation functions are relatively new. In 1999, Petersen et al. showed that bone marrow cells are differentiated to hepatic cells (Petersen B.E. et al., Science 284, 1168(1999)), and in the next year, the fractionated cells obtained by sorting murine hematopoietic cells by c-kit^{high}, Thy-l^{low}, Lin^{neg} and Sca-1⁺ are transdifferentiated to stem cells (Lagasse, E. et al., Nature Medicine 6, 1229-1234(2000)). It is thought that hematopoietic stem cells have transdifferentiation abilities, and differentiation thereof to cardiac muscle (Orlic, D. et al., Nature 410, 701-705(2001)), as well as to alveolar epithelium, intestine epithelium and skin (Orlic, D. et al., *supra*), has been reported.

Thus, although studies on the mesenchymal or ectodermal stem cells have been progressed, the number of reports on endodermal stem cells is small. As for hepatic stem cells, although the existence thereof is thought to be indisputable, there are no reports so far which confirmed the stem cells. As for pancreas, the group of Cornelius et al. separated islet-producing stem cells (IPSCs) from an adult mouse, and reported transplantation experiment of the islet prepared from IPSCs *in vitro* (Ramiya, V.K. et al., Nature Medicine 6, 278-282(2000)). Although differentiation of these cells into α, β and δ cells was confirmed, differentiation potential to other cells has not been confirmed. Although it has been reported that the stem cells separated by collection of nestin-positive cells from islet differentiated to cells having a phenotype of endocrine or exocrine of pancreas, and to cells having a phenotype of liver (Zulewski, H. et al., Diabetes 50, 521-533(2001), immunohistological search by using differentiation markers is not shown.

Induction to hepatic or pancreatic cells from ES cells (embryonic stem cells) has been tried, and differentiation to α or β cells of pancreas can be induced (Lumelsky, N, et al., Science 292, 1389-1394(2001)). However, induction to hepatic cells has not been reported.

As described above, a stem cell capable of being differentiated to hepatic cells has not been obtained. Therefore, a stem cell capable of being differentiated into cells of a plurality of organs including liver has also not been obtained. In case of using stem cells for regenerative medicine, it is most preferred to transplant cells originated from the patient himself in view of preventing the rejection reaction associated in the transplantation. Therefore, if there is a stem cell which can also be prepared from an adult, it is advantageous for regenerative medicine.

### Disclosure of the Invention

An object of the present invention is to provide a stem cell which can be differentiated to a hepatic cell. Another object of the present invention is to provide a stem cell which can be differentiated to cells of a plurality of organs including liver. A still another object of the present invention is to provide a stem cell which can be differentiated to a hepatic cell and which can be prepared also from an adult.

The present inventors intensively studied to succeed in separating a stem cell from salivary gland duct epithelium, which can be differentiated to a hepatic cell, a pancreatic cell and to a salivary gland cell, thereby completing the present invention.

That is, the present invention provides an isolated stem cell originated from salivary gland duct epithelium, which can be differentiated to an alpha-fetoprotein-positive cell, an albumin-positive cell, an amylase-positive cell, an insulin-positive cell and a glucagon-positive cell by culture *in vitro*. The present invention also provides use of a stem cell originated from salivary gland duct epithelium, which can be differentiated to an alpha-fetoprotein-positive cell, an albumin-positive cell, an amylase-positive cell, an insulin-positive cell and a glucagon-positive cell by culture *in vitro*, for the production of cells for transplantation for organ regeneration. The present invention further provides a method for transplanting cells comprising administering an effective amount of stem cells originated from salivary gland duct epithelium to a living organism, which cells can be differentiated to alpha-fetoprotein-positive cells, albumin-positive cells, amylase-positive cells, insulin-positive cells and glucagon-positive cells by culture *in vitro.* The present invention still further provides a method for regenerating an organ, comprising administering an effective amount of stem cells originated from salivary gland duct epithelium to a living organism, which cells can be differentiated to alpha-fetoprotein-positive cells, albumin-positive cells, amylase-positive cells, insulin-positive cells and glucagon-positive cells by culture *in vitro*.

By the present invention, a stem cell which can be differentiated to hepatic cell was first provided. The stem cell according to the present invention can be differentiated not only to the liver, but also to a plurality of organs such as pancreas and salivary gland. The stem cell according to the present invention may be prepared also from an adult. Danger and burden of a patient are much smaller in cases where cells are prepared from salivary gland than in cases where cells are prepared from an organ located in the belly or chest. Thus, the stem cell according to the present invention may easily be prepared from the patient himself who is to receive the transplantation, so that the rejection reaction problematic in transplantation can be easily and surely avoided. Therefore, the stem cell according to the present invention is thought to greatly contribute to regenerative medicine such as regeneration of the liver and the like.

### Brief Description of the Drawing

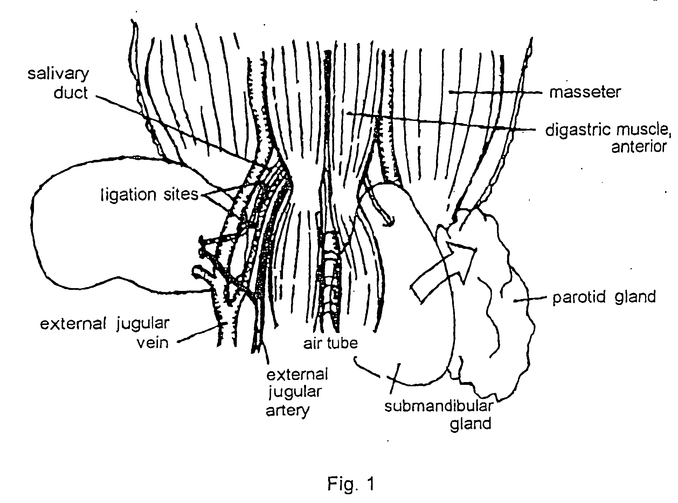

Fig. 1 is a drawing for explaining the method for ligating main discharging duct of submandibular gland, which was carried out when obtaining the stem cells according to the present invention.

### Best Mode for Carrying out the Invention

The stem cell according to the present invention is originated from salivary gland duct epithelium, which was separated from vigorously growing gland epithelium in a submandibular gland which was shrunk by ligation, as will be described in detail in Examples below. Although the stem cells described in Examples below were separated by ligated salivary glands, since the gland epithelium of a normal salivary gland which is not ligated is also growing at least to some degree, the stem cells may also be separated from the normal salivary gland which is not ligated.

The cells according to the present invention have (1) proliferation ability, (2) self-maintaining ability and (3) multipotency, so that they are actual stem cells (Potten, C.S. et al., Development 110, 1001, 1990). Each of the above-mentioned abilities (1), (2) and (3) will now be described in more detail. (1) The doubling time of the cells according to the present invention during logarithmic phase in the culture on a collagen-coated plate is about 18 hours. (2) In culture system, the cells form clusters from day 5 and express various differentiation markers. Even after the group of cells which reached the final differentiation stage is peeled off from the surface of the plate together with the accumulated extracellular matrix, very small number of cells remain on the plate, and the cells start proliferation again. From this phenomenon, it is thought that maintaining the stem cells is attained during induction of differentiation *in vitro*. (3) Although 9 months have passed since the separation of the cells according to the present invention, the cells have not lost multipotency during the subculture for a long time. From these facts, it is apparent that the cell according to the present invention is a stem cell.

When a single cell separated from the gland epithelium of salivary gland is cultured *in vitro* on a plate, although the cell does not express a differentiation marker at the beginning, various cells expressing differentiation markers emerge from the time at which the cells are stacked after confluency. The cells expressing differentiation markers are mainly located in the regions at which the cells are stacked (cord and cluster regions). The differentiation markers expressed are alpha-fetoprotein (AFP), albumin, amylase, insulin and glucagon (needless to say, all of these differentiation markers are not simultaneously expressed in a single cell, but cell groups including a plurality of different cells which express the various differentiation markers, respectively, are generated). Among the above-mentioned markers, AFP and albumin are differentiation markers of the liver, insulin and glucagon are differentiation markers of the pancreas (pancreatic endocrine (islets of Langerhans), and amylase is a differentiation marker of salivary gland and pancreas (exocrine). Thus, the stem cell according to the present invention is a multipotential stem cell which can be differentiated at least to the liver, pancreas and salivary gland. The various cells which respectively express the above-mentioned various differentiation markers are generated by culturing the stem cell according to the present invention under the same normal culturing conditions, and it is not necessary to add a growth factor such as EGF to the culture medium.

In Examples below, the stem cells according to the present invention were obtained from adults. When transplanting cells for regenerative medicine, it is most preferred to transplant the cells of the patient himself in view of prevention of the rejection reaction by the transplantation. Since the stem cell according to the present invention can be obtained from an adult, the cells can be prepared from the patient himself who is to receive the transplantation, which is very advantageous for transplantation. The stem cell according to the present invention may be obtained from not only adults, but also immature infants. Further, in Examples below, although the stem cells according to the present invention were obtained from rats and mice, they may be obtained from other mammals including human, which have salivary glands.

Needless to say, the cells according to the present invention are not limited to the cells separated from salivary gland duct epithelium, but the stem cells obtained during the primary culture or during the subculture, which can generate the cells expressing the above-mentioned various differentiation markers, are within the scope of the present invention.

Since the stem cell according to the present invention can be differentiated to at least the liver, pancreas and salivary gland, these organs may be regenerated by transplanting the stem cells according to the present invention. Transplantation of the stem cells may easily be attained by infusing the suspension of the stem cells. The cells may be infused into the spleen, or into the organ to be regenerated or the vicinity thereof, or into a vein or the like. The number of the stem cells to be infused is not restricted as long as effective for the regeneration of the organ, and may be appropriately selected depending on the symptom, body weight of the host, administration method and the like, and usually about 10² to 10¹⁰ cells. The medium of the cell suspension is preferably a buffer such as physiological phosphate buffer, which is not toxic to the body.

As will be concretely described in Examples below, by administering a suspension of the stem cells to the spleen of an animal in which 2/3 of the liver had been removed; the liver was regenerated. Further, by a genetic test, it was confirmed that the cells originated from the administered stem cells were included in the cells constituting the regenerated liver. Thus, the stem cells according to the present invention can be used at least as the cells for transplantation for the regeneration of the liver. As is evident from the above-mentioned differentiation markers expressed by culturing the stem cell according to the present invention, pancreas cells may be generated from the stem cell according to the present invention. Therefore, the stem cells according to the present invention may be used for the regeneration of pancreas and for the therapy of type I diabetes.

### Examples

The present invention will now be described more concretely by way of examples thereof.

### Example 1 Preparation of Stem Cells

### (1) Method for Growing Salivary Gland Duct Epithelium Cell Fraction in vivo

The main discharging ducts of salivary glands of male rats of 4 to 5 weeks old were ligated as described below. Main salivary glands include three glands, that is, parotid gland, submandibular gland and sublingual gland. Among these glands, submandibular gland which is most evident and which has the largest volume was selected.

Each rat was anesthetized by intraperitoneally administering pentobarbital. After retaining the rat on a dissection table, the neck was extended and median incision was performed on the neck skin. By reversing the skin, the adhered right and left submandibular glands were observed at the median line. The connective tissue enclosing the gland tissue was peeled off, and the right and left submandibular glands were reversed, respectively. By the reversal of the submandibular gland, the cervical dissecting chart shown in Fig. 1 was obtained. Since the main discharging duct of the submandibular gland runs parallel to the external maxillary artery and then to the anterior facial vein branched from the external jugular vein and then submerges under the anterior branch of anterior digastric muscle, the main discharging duct alone was ligated at two sites without touching these large blood vessels. After the ligation, the right and left submandibular glands were restored to the original locations, and the connective tissues enclosing the submandibular glands were sutured, followed by suture of the skin.

On the sixth day from the double ligation of the submandibular ducts of both sides, each rat was sacrificed by bleeding, and the submandibular glands at both sides were enucleated. The submandibular glands in which the main discharging ducts were ligated were apparently shrunk when compared with the submandibular glands of normal control rats of the same time. By histological observation of the shrunk submandibular glands after the ligation, it was observed that most of the acinar cells had dropped and that the gland duct epithelial cells forming the lumina had prominently proliferated.

### (2) Methods for Digesting Salivary Gland Tissues after Ligation Treatment of Ducts and for Separating Cells

The duct-ligated submandibular glands obtained (1) were used. The 10 to 12 submandibular glands (5 to 6 rats) enucleated after sacrifice of the rats were minced into pieces with 2 mm diameters. The minced tissues were transferred to a 50 ml centrifuge tube together with 30 ml of EGTA buffer (containing 8g of NaCl, 0.4 g of KCl, 69 mg of NaH₂PO₄, 75 mg of Na₂HPO₄, 2.38 g of HEPES, 0.19 g of EGTA, 0.35 g of NaHCO₃, 0.9 g of glucose and 6 mg of Phenol Red in IL) and shaken by rotation at a rate of 10 rpm at 37°C for 20 minutes. After the incubation, the minced tissue fluid was centrifuged (100 x g, 5 minutes, room temperature), and the supernatant was discarded. The obtained pellet was dispersed in 60 ml of a digestion medium (containing 60 ml of DMEM/F12:1;1, 100 mg of collagenase and 80 mg of hyaluronidase in 60 ml), and the dispersion was transferred to a 50 ml centrifuge tube, followed by shaking the dispersion by rotation at a rate of 10 rpm at 37°C for 40 minutes. After the incubation, the minced tissue fluid was centrifuged (100 x g, 5 minutes, room temperature), and the supernatant was discarded. The obtained pellet was dispersed in 60 ml of a dispersion medium (containing 60 ml of DMEM/F 12:1;1, and 80 mg (1000U/ml)) of dispase in 60 ml), and the dispersion was transferred to a 50 ml centrifuge tube, followed by shaking the dispersion by rotation at a rate of 10 rpm at 37°C for 60 minutes. During the shaking by rotation, pipetting was performed several times with a 10 ml pipette, thereby mechanically dispersing the minced tissue fluid. After the incubation, the minced tissue fluid was filtered through a cell filter to obtain a cell suspension. The cell suspension was centrifuged (100 x g, 5 minutes, 4°C) to obtain a cell pellet. The cell pellet was suspended in 10 ml of DMEM/F-12, and washed three times with the medium.

The number of cells was counted, and the rate of living cells was checked. The total number of the cells obtained from 10 gland tissues was about 2.0-2.5 x 10⁷. The rate of living cells was not less than 95%.

### (3) Methods for Primary Culture of Salivary Gland Duct Epithelial Cells and for Preparation of Cell Lines

The cell suspension obtained in (2) was placed in a 100 mm dish at a density of 2.0-5.0 x 10⁵ cells/dish, thereby starting primary culture. As the dish for culture, a dish coated with type I collagen was used. As the medium for the primary culture, William's E medium supplemented with 10 ng/ml of recombinant human EGF (epidermal growth factor) , 10% FBS (fetal bovine serum), 10⁻⁸ mol/L of insulin, 10⁻⁶ mol/L of dexamethasone, 100 U/mL of penicillin G and 100 µU/ml of streptomycin was used.

In the cell suspension obtained in (2), the cells were not completely dispersed into single cells, and a number of sites at which cell clusters each of which consists of several cells adhered to the surface of the plate were observed. Cells which did not adhere to the surface of the plate existed, which were thought to be inflammatory cells such as lymphocytes that infiltrated into the salivary gland tissues. All of the non-adhering cells were removed by aspiration when replacing the medium.

Some of the epithelial cell clusters adhered to the surface of the plate exhibited vigorous growth and they formed colonies composed of cells valvately arranged. After the colonies grew to the stage at which each colony consisted of not less than 10 cells, pick up (P2) of colonies using a cloning ring was performed. The cells forming the colonies were washed twice with PBS, and the cells were peeled off from the surface of the plate by the treatment with 0.03% trypsin-EDTA. The picked up cells were transferred to a 24-well plate coated with type I collagen and culture was continued.

The cells proliferated on the entire surfaces of the 24 wells were transferred to a 35 mm dish (P3) and then to 60 mill dish (P4), thereby sequentially increasing the number of cells, and the culture scale was finally expanded to a 100 mm dish (P5). After the scale was expanded to the 100 mm dish, EGF was removed from the medium for primary culture, and the resulting medium was used thereafter as a maintenance medium. No problem was caused in the culture even after the removal of EGF.

Single cell culture was performed using the epithelial cells proliferated on the 100 mm dish. The gland duct epithelial cells were peeled off with 0.03% trypsin-EDTA, and mechanically dispersed by pipetting, and then filtered through a cell filter (pore size: 40 µm). Unlike the time of the primary culture, the cells were dispersed into single cells. The cells were plated after dilution to 1.0 x 10² cells/100 mm dish, and it was confirmed that each cell adhered to the plate in the form of a single cell. Although a part of the cells dropped, several sites at which colonies were formed were also observed. Cells were picked up from these colony-forming sites, and culture of the cells was continued on a 35 mm dish. The culture scale was expanded subsequently, thereby establishing cell lines. Two cell lines were obtained by the method described above. Among the obtained two cell lines (A line and B line), subculture was carried out from the N line to obtain four subclones, SN-1 (subculture of N line-1) to SN-4.

### Example 2 Properties of Cells

Each of the cell lines obtained in Example 1 was plated on a 100 m dish at a density of 2 x 10⁵ cells/dish, and three days after (day 3), the cells proliferated in a single layer to reach confluency (1.0 x 10⁶ cells/dish). After the confluency (day 5-), the cells started to stack in various regions in the monolayer and regions of bilayer (cord) were started to be formed. Further, at some of the cords, cell clusters were formed. Thereafter, monolayer cells gradually dropped. Although the cords and clusters remained for some time, these also gradually dropped after day 15, and were completely peeled off after day 30. After all of the cords and clusters were peeled off from the plate, only a small number of cells remained on the dish. By culturing these cells continuously, they again proliferate to form colonies and then to form a monolayer. Thereafter, the formation of cords and clusters was observed as in the culture for the first time, and this cycle was repeated thereafter.

The markers expressed by the cells were examined by immunocytostaining or flow cytometry. All of the fluorescence-labeled antibodies used in the staining were commercially available, and the protocol of the method was in accordance with the instructions attached to the commercially available fluorescence-labeled antibodies or the commercially available flow cytometry apparatus. That is, immunocytostaining was carried out in accordance with the immunohisto/cytostaining guide by DAKO. The flow cytometry was performed in accordance with FCM staining protocol of BD Phermingen. A list of the used antibodies is shown below. It was confirmed that all of the antibodies which were not described as "anti-rat" reacted with rat from the information from the manufacturer or papers.

### List of Antibodies

Rabbit anti-human AFP polyclonal antibody (A008. code number), produced by DAKO
Mouse anti-rat insulin/proinsulin monoclonal antibody (5E4/3), produced by Biogenesis
Mouse anti-rat integrin α6β1 monoclonal antibody (MAB1410. Catalog number), produced by Chemicon
Goat anti-human amylase polyclonal antibody (C-20), produced by Santa Cruz
Mouse anti-rat CD34 monoclonal antibody (ICO115), produced by Santa Cruz
Rabbit anti-c-kit polyclonal antibody (H-300), produced by Santa Cruz
Rabbit anti-rat albumin polyclonal antibody, produced by ICT
Rabbit anti-human glucagon polyclonal antibody (A0565), produced by DAKO
Anti-CK19 monoclonal antibody (catalog number NCL-CK19/ clone b170), produced by Novocastra Laboratories Ltd
Anti-rat nestin monoclonal antibody (catalog number 556309/ clone Rat401), produced by BD PharMuyen
Anti-rat CD45 monoclonal antibody (catalog number 22134D/ clone OX-1), produced by BD PharMingen
Anti-rat Thy-1 monoclonal antibody (catalog number 22212D/ clone OX-7), produced by BD PharMingen
Anti-rat laminin polyclonal antibody (/catalog number Z0097/), produced by DAKO

Immunocytostaining was performed on the cells after subculturing 20 times. As a result, although albumin-positive cells were observed on day 5- after plating, CK-19-positive cells were not observed, which is a representative marker of hepatic epithelial cells (oval cells). Amylase-positive cells, which is a marker of exocrine cells of salivary duct (and pancreas), were also observed after day 5 similar to albumin-positive cells. Some acinar structures composed of a plurality of amylase-positive cells were also observed. As for insulin and glucagon, which are pancreatic endocrine markers, which were also examined, positive cells were observed. In double staining with insulin and glucagon, formation of clusters in which the cells that were positive to these markers was observed. AFP-positive cells, which is a marker of potential stem cell of hepatocyte, were also observed. Nestin-positive cells, which is thought to be a marker of pancreatic stem cell, were also observed. All of the cells positive to these markers were localized to the cords and clusters. These positive cells were observed in all of the above-described 4 subclones after day 5 (day 5-) from the plating. Based on these facts, it is thought that each of the all cells separated from the salivary gland duct epithelia by the above-described is multipotent.

The markers reported as existing on the hepatic epithelial cells and hepatic precursor cells (in regenerating liver), which are candidates of hepatic stem cells, were also examined. The cells at 12 to 24 hours after the plating were examined by immunocytostaining and flow cytometry. As a result, expression of VLA-6 (α6β1 integrin) reported to exist on hepatic precursor cells was confirmed. Expression of laminin which is a ligand of VLA-6 was also observed. Expression of AFP or CK19 which are representative markers of hepatic epithelial cells, was not observed. Surface antigens reported to exist on hematopoietic cells and hepatic epithelial cells were also examined. As a result, the percentage of Thy-1⁺ cells was 2.5%, and that of c-kit⁺ cell was about 5%. As for CD34, c-kit⁺ cell population was simultaneously weakly positive to CD34. CD45-positive cells did not exist at all.

Whether the cells obtained in Example I exhibit characters of side-population (SP) known as a character of tissue stem cells, staining with Hoechst 33342 (trademark) was carried out by a conventional method. As a result, existence of SP fraction was confirmed. Although complete disappearance of the SP fraction was not observed in the presence of 50 µM veraparnil, increase of intensity of Hoechst 33342 was observed in the Hoechst 33342-positive cell population. From these facts, it was proved that the cells according to the present invention are verapamil-sensitive with respect to staining with Hoechst 33342, and Hoechst dye efflux component exists, which is a feature of SP cells.

### Example 3 Transplantation of Cells to Living Organism (Part 1)

The cell suspension prepared in Example 1(2), obtained by digesting the salivary glands after ligating the ducts was transplanted to a female rat. In accordance with the 2AAF/PH protocol, 2 mg/day of 2-acetylaminofluorene was started to be administered from 6 days before the cell transplantation, and the cell transplantation was performed after excision of 2/3 of the liver. The cell transplantation was performed by infusing the cell suspension to the spleen of the recipient (the animal to receive the transplantation). The total number of infused cells was about 8.0 x 10⁶ cells. Detection of the cells from the donor in the liver of the recipient was performed by *in situ* hybridization using the SRY gene in the Y chromosome as a DNA probe, according to a conventional method. Since the recipient was a female rat and the donors were male rats, the genes existing on the Y chromosome do not exist in the recipient and exist only in the donors. Therefore, if the SRY gene is detected in a cell, it is proved that the cell is originated from the donor. This cross-sex transplantation was carried out in two models, that is, from male SD rats to a female SD rat, and from male LEA rats to a female LEC rat.

As a result, in both of the transplantation models, in the second week after the transplantation, ductile structures were formed in the vicinity of the portal triad region, and atypical ductular proliferation of the cells having oval nuclei was observcd at various regions. These cells were CK19-positive, and are thought to be hepatic epithelial cells. By the *in situ* hybridization of the SRY gene at the same regions, the signal was observed in a part of the cells which were thought to be originated from the donor. In the bile duct epithelial cells and in a very small number of the hepatic cells, SRY signal was observed. In the hepatic tissue at four weeks after the transplantation, most of the hepatic epithelial cells had disappeared. SRY-positive cells were unchangeably observed in the bile duct epithelial cells and in a part of the hepatic cells. Thus, in the regenerated liver of the recipient, existence of the cells originated from the salivary gland epithelia of the donors was confirmed. Morphologically normal cells were observed in the epithelial cells at one to two weeks after the transplantation, and were observed in the bile duct epithelial cells and hepatic cells after two weeks after the transplantation. By histological examination up to 5 weeks after the transplantation, tumor cells exhibiting dyskaryotic character or the like were not observed. By double staining by *in situ* hybridization and immunohistochemistry, it was confirmed that a part of the anti-rat albumin-positive cells were SRY gene-positive. The immunohistostaining was performed by using the above-mentioned commercially available rabbit anti-rat albumin polyclonal antibody in accordance with the immunohisto/cytostaining guide by DAKO.

As described above, it was proved that stem cells which were differentiated to hepatic cells existed in the salivary gland tissues after ligation of the ducts, prepared in Example 1(2).

### Example 4 Transplantation of Cells to Living Organism (Part 2)

A suspension of the cells obtained after subculture for a long period (three months) prepared in Example 1(3) was prepared. The cell suspension was prepared by treating the monolayer cells proliferated on a type 1 collagen-coated plate for 3 to 4 days, with trypsin-EDTA (0.05% trypsin and 0.53 mM EDTA), and by peeling off the cells from the surface of the plate, followed by dispersing the cells. The cell suspension was administered to a female rat as in Example 3. The total number of the administered cells was about 1.0 x 10⁶.

As a result, as in Example 3, differentiation into hepatic epithelial cells, bile duct cells and hepatic cells was confirmed. By the macroscopic and microscopic examinations up to 5 weeks after the transplantation, emergence of cells exhibiting tuberculation or dyskaryotic character was not observed. By double staining by *in situ* hybridization and immunohistochemistry, it was confirmed that a part of the antiral albumin-positive cells were SRY gene-positive. From these observations, it was thought that the stem cells according to the present invention have potency to differentiate into functional hepatic cells having albumin-producing ability even after culturing for a long time.

### Example 5 Transplantation of Cells to Living Organism (Part 3)

The same operations as in Examples 1(1)(2) and 3 were repeated except that mice were used in place of rats. The mice subjected to the ligation of the main discharging ducts of the submandibular glands were male C57BL/6NCrj(commercially available from Charles River Japan Inc). Six days after the ligation of the ducts, ductal proliferation and disappearance of the gland cells were confirmed. As the recipient in the transplantation experiment, a female C57BL/6 mouse was used. As in Example 3, detection of the donor cells was carried out by detecting the sry gene on the Y chromosome. That is, since the sry gene does not exist in the cells of the recipient, but exist in the cells of the donors. if a cell is sry gene-positive, it is proved that the cell is originated from the donor.

In the liver at two weeks after the transplantation, sry gene-positive donor cells were observed. These results were the same as those of Examples 3 and 4, and it was proved that multipotential stem cells also exist in mouse.

## Claims

1. An isolated stem cell originated from salivary gland duct epithelium, which can be differentiated to an alpha-fetoprotein-positive cell, an albumin-positive cell, an amylase-positive cell, an insulin-positive cell and a glucagon-positive cell by culture *in vitro*.

2. The stem cell according to claim 1, which is originated from an adult.

3. Use of a stem cell originated from salivary gland duct epithelium, which can be differentiated to an alpha-fetoprotein-positive cell, an albumin-positive cell, an amylase-positive cell, an insulin-positive cell and a glucagon-positive cell by culture *in vitro*, for the production of cells for transplantation for organ regeneration.

4. The use according to claim 3, wherein said organ is liver or pancreas.

5. The use according to claim 4, wherein said organ is liver.

6. The use according to any one of claims 3 to 5, wherein said stem cell is originated from the individual who is to receive the transplantation.

7. A method for transplanting cells comprising administering an effective amount of stem cells originated from salivary gland duct epithelium to a living organism, which cells can be differentiated to alpha-fetoprotein-positive cells, albumin-positive cells, amylase-positive cells, insulin-positive cells and glucagon-positive cells by culture *in vitro*.

8. The method according to claim 7, wherein said stem cells are originated from the individual who is to receive the transplantation.

9. A method for regenerating an organ, comprising administering an effective amount of stem cells originated from salivary gland duct epithelium to a living organism, which cells can be differentiated to alpha-fctoprotein-positive cells, albumin-positive cells, amylase-positive cells, insulin-positive cells and glucagon-positive cells by culture *in vitro*.

10. The method according to claim 9, wherein said organ is liver or pancreas.

11. The method according to claim 10, wherein said organ is liver.

12. The method according to any one of claims 9 to 11, wherein said stem cells are originated from the individual who is to receive the transplantation.
